# EUROPÄISCHE PATENTSCHRIFT

(11) **EP 2 037 992 B1**
(45) Veröffentlichungstag und Bekanntmachung des Hinweises auf die Patenterteilung: **11.01.2012**
(21) Anmeldenummer: 07785941.1
(22) Anmeldetag: 09.07.2007
(51) Int. Cl.: A61M 15/00

(54) **VORRICHTUNG ZUR DOSIERTEN ABGABE VON VERSPRÜHBAREN STOFFEN**
DEVICE FOR DOSED ADMINISTRATION OF SPRAYABLE SUBSTANCES
DISPOSITIF DE DÉLIVRANCE DOSÉE DE MATIÈRES PULVÉRISABLES

(30) Priorität: 11.07.2006 DE 102006032293
(43) Veröffentlichungstag der Anmeldung: 25.03.2009
(73) Patentinhaber: Friedrich Sanner GmbH & Co. KG, 64625 Bensheim (DE)
(72) Erfinder: LANG, Sven, Markus, 76669 Bad Schönborn (DE); TIESBERGER, Kai, 65527 Niedernhausen (DE)
(74) Vertreter: Reiser, Tonio Andreas
(86) Internationale Anmeldenummer: PCT/EP2007/006052
(87) Internationale Veröffentlichungsnummer: WO 2008/006527

(56) Entgegenhaltungen:
- WO-A-02/24269
- WO-A-02/36190
- US-A- 5 411 173
- US-A- 5 575 280
- US-A- 5 743 252

## Beschreibung

Die vorliegende Erfindung betrifft eine Vorrichtung zur dosierten Abgabe von versprühbaren Stoffen, insbesondere von Aerosolen, mit einer elektronischen Einrichtung zur Erfassung der Abgaben, und mit einem ersten Gehäuseteil, der eine Aufnahme für einen den versprühbaren Stoff enthaltenden, mit einem Ventil versehenen Behälter bildet.

Solche Vorrichtungen zur dosierten Abgabe von versprühbaren Stoffen können insbesondere als Inhalatoren ausgebildet und in der Medizin zur Behandlung von Krankheiten eingesetzt werden. Derartige Inhalatoren eignen sich z.B. zur Behandlung von Asthma, Allergien oder auch anderen Krankheiten und dienen insbesondere der dosierten Verabreichung eines Medikaments, welches über Mund oder Nase aufgenommen wird. In diesem Zusammenhang spricht man auch von sogenannten Dosier-Aerosolen. Gerade bei lebensbedrohlichen Krankheiten ist es wichtig, dass der Verwender Informationen über die in dem Behälter enthaltene Restmenge verfügt. Optisch ist dies in der Regel nicht zu erkennen, da der den versprühbaren Stoff aufnehmende Druckbehälter meist aus einem nicht durchsichtigen Material besteht. Hieraus ergibt sich die Bedeutung einer elektronischen Einrichtung zur Erfassung der erfolgten Abgaben des versprühbaren Stoffes. Durch diese erhält der Verwender eine zuverlässige Information über die abgegebene bzw. die in dem Behälter verbleibende Menge des versprühbaren Stoffes und kann dafür sorgen, stets mit einem ausreichenden Vorrat des jeweiligen versprühbaren Stoffes versorgt zu sein.

Vorrichtungen zur dosierten Abgabe von Medikamenten sind in unterschiedlicher Ausgestaltung bekannt. So zeigt die EP 0 448 204 B1 eine solche Vorrichtung mit einem einteiligen Gehäuse, in dem eine Aufnahme für einen den versprühbaren Stoff aufnehmenden Behälter vorgesehen ist. Das Gehäuse weist dabei ein winklig zu der Behälterlängsachse angeordnetes hohles Mundstück auf, über das der versprühbare Stoff bei Betätigung eines an dem Behälter vorgesehenen Ventils abgegeben und inhaliert werden kann. Die Abgabe des versprühbaren Stoffes erfolgt dadurch, dass der Behälter auf das Gehäuse zu bewegt und dabei das an dem Behälter vorgesehene Ventil betätigt wird. Zur Erfassung der Anzahl der Abgaben ist die vorbekannte Vorrichtung mit einer elektronischen Einrichtung versehen, welche einen Schalter aufweist, der direkt an dem Behälter anliegt und von diesem betätigt wird.

Ein weiterer Inhalator ist in der EP 0 775 499 B1 beschrieben. Hier ist auf dem oberen Gehäuseende eine Vorrichtung angeordnet, die die Anzahl der dosierten Abgabemengen anzeigen soll.

Bei der in der US 6,138,669 beschriebenen Inhalator wird die Anzahl der Abgaben über einen Sensor ermittelt, der den Druckstoß in dem Verbindungskanal des Mundstückes misst.

Die US 5,411,173 beschreibt eine elektronische Zähleinrichtung für eine Sprayvorrichtung, mit der die Anzahlt der Abgaben erfasst und auf einem Display angezeigt werden kann. Die vorbekannte Vorrichtung kann mittels eines Clips an der Seitenwand des Sprayergehäuses befestigt werden. Die Zähleinrichtung wird durch einen Stiel betätigt, welcher beweglich gehalten ist. Hierzu kann ein Fußabschnitt vorgesehen sein, welcher an der Unterseite des die Flüssigkeit aufnehmenden Behälters anliegt.

Vor diesem Hintergrund stellt sich die Erfindung die Aufgabe, eine Vorrichtung anzugeben, welche einfach zu bedienen ist und die Anzahl und/oder Dauer der Abgaben des versprühbaren Stoffes zuverlässig ermittelt.

Diese Aufgabe wird bei einer gattungsgemäßen Vorrichtung zur dosierten Abgabe mit den Merkmalen von Anspruch 1 gelöst

Die erfindungsgemäße Vorrichtung ist vorteilhaft. So erfolgt die Erfassung der Abgabe des versprühbaren Stoffes unabhängig von dem Behälter, da der Schalter durch die Relativbewegung von erstem und zweitem Gehäuseteil betätigt wird. Daher kann die Vorrichtung auch unabhängig von der genauen Form und/oder Größe des jeweils eingesetzten Behälters funktionieren, so dass ein und dieselbe Vorrichtung in Verbindung mit verschiedenen Behältern eingesetzt werden kann. Dabei kann die Abgabe des versprühbaren Mediums stets zuverlässig erfasst werden.

Der versprühbare Stoff kann vorzugsweise als eine Flüssigkeit oder ein Pulver vorliegen. Der versprühbare Stoff kann auch eine Lösung und/oder eine Mischung verschiedener Substanzen umfassen. Insbesondere kann der versprühbare Stoff eine therapeutisch wirksame Zusammensetzung, wie ein Medikament sein.

Indem erster und zweiter Gehäuseteil in der Freigabeposition nicht derart axial zueinander beweglich sind, dass das Ventil durch den zweiten Gehäuseteil betätigt werden kann, werden Fehlauslösungen beim Zusammenbau, z.B. nach der Reinigung vermieden.

Eine Verbesserung wird dadurch erreicht, dass der erste und der zweite Gehäuseteil insbesondere über einen Bajonettverschluss derart verbunden sind, dass erster und zweiter Gehäuseteil zueinander eine Arbeitsposition und eine Freigabeposition einnehmen können, wobei in der Arbeitsposition der erste und zweite Gehäuseteil relativ zueinander in axialer Richtung beweglich sind und wobei in der Freigabeposition der zweite Gehäuseteil von dem ersten Gehäuseteil abnehmbar ist. Auf diese Weise können in der Arbeitsposition die beiden Gehäuseteile aufeinander zu bewegt werden, um das Ventil des Behälters zu betätigen. In der Freigabeposition kann der zweite Gehäuseteil z.B. zur Reinigung abgenommen werden.

Nach einer vorteilhaften Ausgestaltung der Erfindung ist vorgesehen, dass der Schalter ein mechanischer Schalter, ein Näherungsschalter, ein Magnetschalter, und/oder ein optischer Schalter ist. Auf diese Weise wird ein elektrisches Signal erzeugt, welches es ermöglicht, die Abgabe des versprühbaren Mediums zu erfassen. Ein mechanischer Schalter, welcher einen elektrischen Kontakt öffnet bzw. schließt, ist dabei besonders kostengünstig herstellbar und eignet sich besonders für ein Einwegprodukt.

Eine weitere Verbesserung wird dadurch erreicht, dass der Schalter nicht zur Anlage an dem Behälter ausgebildet ist. Die Betätigung des Schalters ist dann von dem Behälter, insbesondere dessen Größe oder Form vollständig unabhängig.

Erfindungsgemäß kann weiter vorgesehen sein, dass in der Aufnahme der Behälter, welcher einen Behälterkörper und einen Behälterkopf mit dem Ventil zur dosierten Abgabe des versprühbaren Stoffes aufweist, zumindest teilweise aufnehmbar ist. Dabei kann der erste Gehäuseteil einen hohlen Schaft aufweisen, in dem der Behälterkörper ganz oder teilweise aufnehmbar ist.

Das Inhalieren eines versprühten Stoffes wird dadurch erleichtert, dass der zweite Gehäuseteil einen ersten Abschnitt zur Aufnahme des Behälterkopfes und einen insbesondere winklig zu dem ersten Abschnitt verlaufenden hohlen zweiten Abschnitt aufweist. Dabei hat sich bewährt, wenn der hohle zweite Abschnitt als ein Mundstück und/oder Nasenstück ausgebildet ist.

Insbesondere die Reinigung der Vorrichtung wird erleichtert, wenn der zweite Gehäuseteil abnehmbar ist. Dann ist es möglich, diesen Gehäuseteil nach dem Abnehmen z.B. separat unter fließendem Wasser zu reinigen.

Vorteilhafterweise werden durch die elektronische Einrichtung zur Erfassung der Abgaben die Anzahl und/oder die Dauer der Abgaben erfasst. Sofern es sich bei dem Behälter um einen solchen mit einem Dosierventil handelt, das bei jeder Betätigung eine gleichbleibende Menge des Stoffes abgibt, kann bereits aus der Anzahl der Betätigungen auf die in dem Behälter verbleibende Restmenge geschlossen werden.

Wenn die elektronische Einrichtung zur Erfassung der Abgaben eine elektronische Recheneinheit und einen elektronischen Datenspeicher aufweist, können die durch den Schalter erfassten Abgaben besonders einfach gezählt werden. Hieraus kann dann die Einrichtung ohne weiteres die in dem Behälter verbleibende Restmenge ermitteln, sofern vorher eine Information über die maximale Anzahl an Abgaben gespeichert wurde.

Nach einer besonders vorteilhaften Ausgestaltung der Erfindung ist vorgesehen, dass die elektronische Einrichtung zur Erfassung der Abgaben eine optische Anzeige aufweist, durch die eine Information über die Anzahl der erfolgten Abgaben und/oder die Anzahl der verbleibenden Abgaben angezeigt werden kann. Als Anzeige kann z.B. eine kostengünstige LCD-Anzeige oder eine andere Anzeige eingesetzt werden.

Eine weitere Verbesserung wird dadurch erreicht, dass die elektronische Einrichtung zur Erfassung der Abgaben einen akustischen Signalgeber aufweist. In diesem Fall kann dem Verwender z.B. eine Fehlbedienung mitgeteilt werden. Denkbar ist auch, einen solchen Signalgeber einzusetzen, um den Verwender an den nächsten Zeitpunkt für die Einnahme des Stoffes zu erinnern.

Von Vorteil ist die elektronische Einrichtung zur Erfassung der Abgaben mit dem Schalter an oder in dem ersten oder zweiten Gehäuseteil angeordnet und wird der Schalter durch ein an dem jeweils anderen Gehäuseteil vorgesehenes Betätigungselement bei einer Relativbewegung von erstem und zweitem Gehäuseteil betätigt. Vorzugsweise ist die elektronische Einrichtung an oder in dem ersten Gehäuseteil angeordnet, da dann der zweite Gehäuseteil leicht gereinigt werden kann, ohne dass hierbei die elektronisch Einrichtung beeinträchtigt wird.

Erfindungsgemäß kann weiterhin vorgesehen sein, dass die Vorrichtung als Inhalationsvorrichtung insbesondere für ein Medikament ausgebildet ist.

Bei dem fertigen Produkt kann vorgesehen sein, dass in der Aufnahme ein mit dem versprühbaren Stoff wenigstens teilweise befüllter Behälter aufgenommen ist. Hierbei kann der versprühbare Stoff vorzugsweise ein Medikament oder eine andere therapeutisch wirksame Zusammensetzung sein.

Die Abgabe des versprühbaren Stoffes ist dann besonders einfach, wenn der Stoff unter Überdruck in dem Behälter aufgenommen ist.

Die Dosierung ist dann einfach und genau, wenn das Ventil des Behälters als ein Dosierventil ausgebildet ist, welches bei jeder Betätigung eine gleichbleibende Menge des versprühbaren Stoffes insbesondere stoßartig abgibt.

Weitere Ziele, Merkmale, Vorteile und Anwendungsmöglichkeiten der vorliegenden Erfindung ergeben sich aus der nachfolgenden Beschreibung eines Ausführungsbeispiels anhand der Zeichnungen. Dabei bilden alle beschriebenen und/oder bildlich dargestellten Merkmale für sich oder in beliebiger Kombination den Gegenstand der Erfindung, auch unabhängig von der Zusammenfassung in einzelnen Ansprüchen oder deren Rückbeziehung.

Es zeigen:
- Fig. 1:: eine perspektivische Darstellung einer erfindungsgemäßen Vorrichtung zur dosierten Abgabe von versprühbaren Stoffen;
- Fig. 2:: eine Draufsicht auf die Vorrichtung aus Fig. 1;
- Fig. 3:: einen Schnitt durch die Vorrichtung aus Fig. 2 entlang der Linie A - A:
- Fig. 4:: eine Explosionsdarstellung der Vorrichtung aus Figur 1;
- Fig. 5:: einen Schnitt durch die Vorrichtung aus Fig. 1 entlang des Schalters in der nicht betätigten Position;
- Fig. 5a:: eine vergrößerte Darstellung des Details K aus Fig. 5;
- Fig. 6:: einen Schnitt durch die Vorrichtung aus Fig. 1 entlang des Schalters in der betätigten Position;
- Fig. 6a:: eine vergrößerte Darstellung des Details J aus Fig. 6;
- Fig. 7:: eine perspektivische Darstellung des ersten Gehäuseteils und der elektronischen Einrichtung zur Erfassung der Abgaben des versprühbaren Stoffes der Vorrichtung aus Fig. 1 vor dem Zusammenbau;
- Fig. 8:: eine perspektivische Darstellung des ersten Gehäuseteils und der elektronischen Einrichtung zur Erfassung der Abgaben des versprühbaren Stoffes der Vorrichtung aus Fig. 1 nach dem Zusammenbau;
- Fig. 9:: eine Teil-Schnittdarstellung des ersten Gehäuseteils aus Fig. 8 mit der elektronischen Einrichtung zur Erfassung der Abgaben des versprühbaren Stoffes;
- Fig. 10:: die Vorrichtung aus Fig. 1, wobei erster und zweiter Gehäuseteil zueinander verdreht sind;
- Fig. 11:: die Vorrichtung aus Fig. 10, wobei der zweite Gehäuseteil abgenommen ist.
- Fig. 12:: einen Schnitt durch die Vorrichtung aus Fig. 1 in der Arbeitsposition;
- Fig. 12a:: einen Schnitt entlang der Linie E-E aus Fig. 12;
- Fig. 13:: einen Schnitt durch die Vorrichtung aus Fig. 10 in der Freigabeposition;
- Fig. 13a:: einen Schnitt entlang der Linie F-F aus Fig. 13;

Die Figuren zeigen eine Vorrichtung zur dosierten Abgabe von versprühbaren Stoffen, welche als ein Inhalator ausgebildet ist. Mit diesem Inhalator können flüssige oder feste Stoffe insbesondere als Aerosole über die Atemwege verabreicht werden. Als versprühbare Stoffe können durch die Vorrichtung insbesondere Medikamente oder andere therapeutisch wirksame Substanzen abgegeben werden.

Die in den Figuren dargestellte Vorrichtung weist ein erstes Gehäuseteil 1 und zweites Gehäuseteil 2 auf. In dem ersten und zweiten Gehäuseteilen 1, 2 ist ein Behälter 3 aufgenommen, welcher den versprühbaren Stoff enthält.

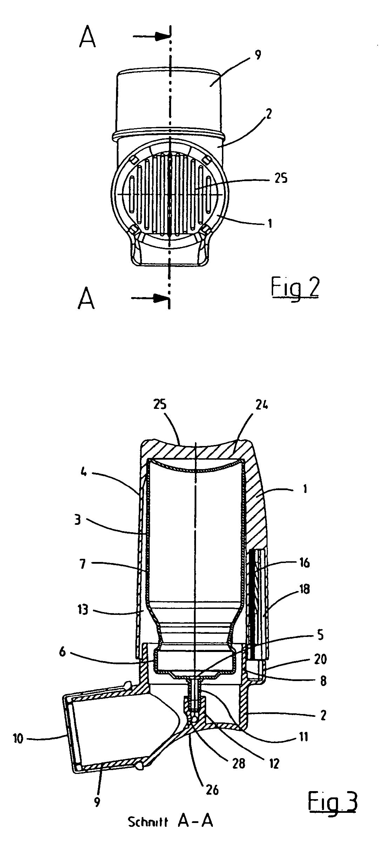

Insbesondere die Figuren 3 bis 6a lassen erkennen, dass der Behälter 3 einen mit einem nicht näher dargestellten Ventil 5 versehenen Behälterkopf 6 und einen Behälterkörper 7 aufweist. Der Behälter 3 ist dabei als ein Druckbehälter aus Metall ausgeführt, in dem der versprühbare Stoff unter Überdruck aufgenommen ist. Aufgrund dieses Überdrucks kann der versprühbaren Stoff bei Betätigung des Ventils 5 selbsttätig abgegeben werden.

Das Ventil 5 ist als Dosierventil ausgebildet, welches bei der Betätigung jeweils eine gleichbleibende Menge des versprühbaren Stoffes als Sprühstoß dosiert abgibt. Auf diese Weise kann der Verwender den versprühbaren Stoff einfach durch ein- oder mehrmaliges Betätigen des Ventils 5 dosieren. Das Ventil 5 weist einen vorstehenden hohlen Ventilstift 11 auf, durch den das Ventil 5 betätigt werden kann. Dabei wird das Ventil 5 durch eine nicht dargestellte Feder in der Geschlossen-Stellung gehalten und kann durch Druck auf den beweglichen Ventilstift 11 in die Offen-Stellung bewegt werden.

Der erste Gehäuseteil 1 bildet eine Aufnahme 13 für den Behälterkörper 7 und weist hierzu einen länglichen hohlen Schaft 4 auf. Figur 3 lässt erkennen, dass der gesamte Behälterkörper 7 in der zylindrischen Aufnahme 13 aufgenommen ist. Hierbei liegt der Behälterkörper 7 an dem ersten Gehäuseteil 1 an. Der erste Gehäuseteil 1 weist an seinem einen Ende einen Bodenabschnitt 24 auf, der mit seiner Außenseite einen ersten Griffabschnitt 25 bildet. An dem gegenüberliegenden Ende ist der erste Gehäuseteil 1 offen.

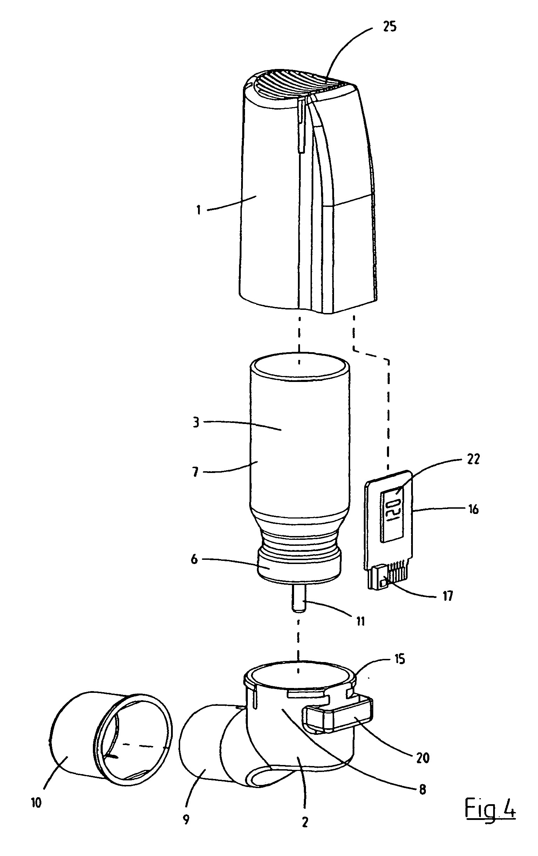

Der zweite Gehäuseteil 2 weist einen ersten Abschnitt 8 zur Aufnahme des Behälterkopfes 6 auf und ist relativ zu dem ersten Gehäuseteil 1 in der Längsrichtung des Ventilstiftes 11 bewegbar. In dem ersten Abschnitt 8 ist ein Betätigungsabschnitt 12 für das Ventil 5 ausgebildet. Der Betätigungsabschnitt 12 liegt an dem Ventilstift 11 an und nimmt das freie Ende des Ventilstifts 11 auf. Ein zweiter Abschnitt 9 des zweiten Gehäuseteils 2 ist hohl ausgebildet und winklig zu dem ersten Abschnitt 8 ausgerichtet. Der hohle zweite Abschnitt 9 bildet bei der dargestellten Ausführungsform ein Mundstück. Alternativ kann der zweite Abschnitt 9 z.B. auch als Nasenstück ausgebildet sein, wenn die Abgabe des versprühbaren Stoffes in die Nase erfolgen soll. Der hohle zweite Abschnitt 9 wird bei Nichtgebrauch durch eine abnehmbare Kappe 10 geschützt, wie den Figuren 3 und 4 entnommen werden kann. Der zweite Gehäuseteil 2 weist zudem dem ersten Griffabschnitt 25 des ersten Gehäuseteils 1 gegenüberliegend einen zweiten, gewölbten Griffabschnitt 26 auf.

Bei Druck auf die beiden gegenüberliegenden Griffabschnitte 25, 26 wird der zweite Gehäuseteil 2 auf den ersten Gehäuseteil 1 zu bewegt und dabei der Ventilstift 11 durch den Betätigungsabschnitt 12 verschoben, wodurch das Ventil 5 geöffnet wird. Eine Dosis des versprühbaren Stoffes tritt jetzt durch den hohlen Ventilstift 11 aus und wird durch eine in dem Betätigungsabschnitt 12 ausgebildete Bohrung 28 so umgeleitet, dass der versprühbare Stoff aus dem hohlen Abschnitt 9 austreten kann. Anschließend kann der Druck auf die Griffabschnitte 25, 26 reduziert bzw. weggenommen werden, worauf erster und zweiter Gehäuseteil 1, 2 unter Wirkung der Feder des Ventils 5 in die Ausgangsposition zurückkehren. Um die Rückkehr in die Ausgangsposition zu erleichtern, kann auch ein nicht dargestelltes Federelement direkt zwischen erstem und zweitem Gehäuseteil 1, 2 vorgesehen werden.

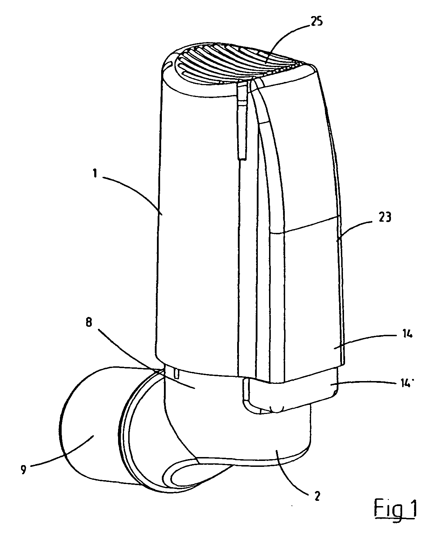

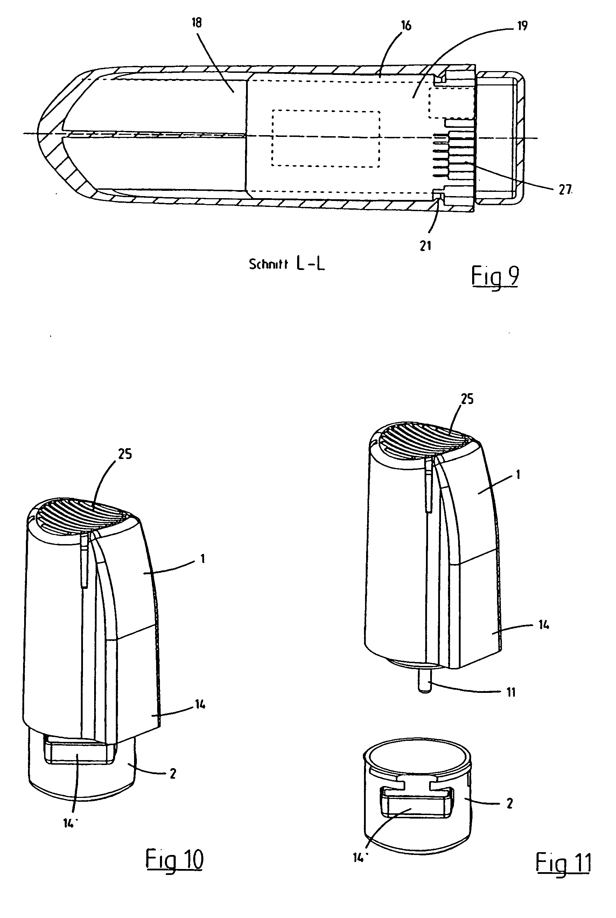

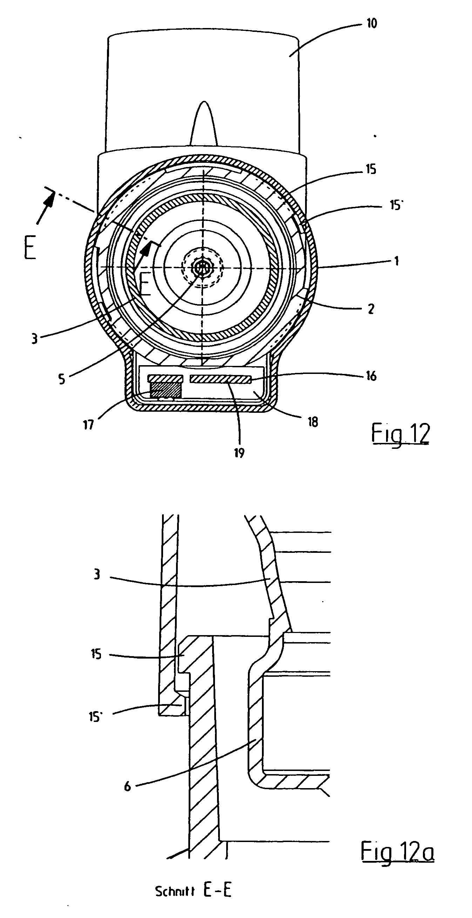

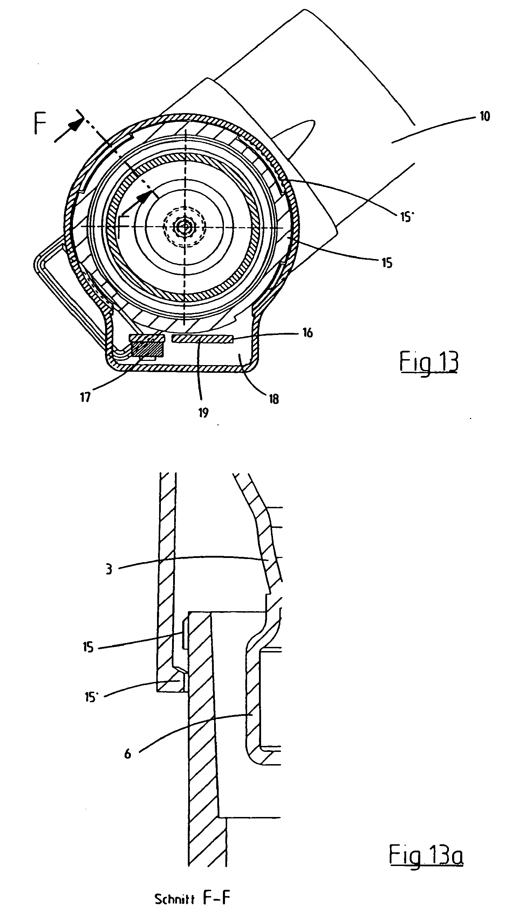

Zur besseren Reinigung kann der zweite Gehäuseteil 2 von dem ersten Gehäuseteil 1 abgenommen werden. Während die Figuren 1, 3 und 12 das erste und zweite Gehäuseteil 1, 2 in ihrer Arbeitsposition zeigen, ist in den Figuren 10 und 13 eine Freigabeposition dargestellt. Erster und zweiter Gehäuseteil 1, 2 können gegeneinander verdreht und dadurch von der Arbeitsposition in die Freigabeposition bewegt werden. Hierzu sind erster und zweiter Gehäuseteil 1, 2 über einen Bajonettverschluss verbunden, der es erlaubt, den zweiten Gehäuseteil 2 in der Freigabestellung von dem ersten Gehäuseteil 1 abzunehmen. Dies ist besonders gut in den Figuren 10 bis 13 zu erkennen, wobei Figur 12 die Arbeitsposition und Figuren 10 und 13 die Freigabeposition darstellen. Nur in der Arbeitsposition können erstes und zweites Gehäuseteil 1, 2 in axialer Richtung zueinander bewegt und damit das Ventil 5 betätigt werden. In der Freigabeposition ist dies nicht möglich, da die Gehäuseteile Anschläge 14, 14' aufweisen, welche eine axiale Bewegung aufeinander zu nur in der Arbeitsposition gestatten. Dadurch können unbeabsichtigte Auslösungen während des Zusammenbaus vermieden werden.

Figur 12a zeigt ein vergrößertes Detail (Schnitt entlang der Linie E-E aus Figur 12) des Bajonettverschlusses. Hier ist gut zu erkennen, dass Vorsprünge 15, 15' des Bajonettverschlusses in der Arbeitsposition verhindern, dass der zweite Gehäuseteil 2 von dem ersten Gehäuseteil 1 abgenommen werden kann. Gleichwohl sind erster und zweiter Gehäuseteil 1,2 so gestaltet, dass diese zur Betätigung des Ventils 5 aufeinander zu bewegt werden können. Figur 13a veranschaulicht, wie in der Freigabestellung, wenn erstes und zweites Gehäuseteil relativ zueinander wie dargestellt verdreht sind, die Vorsprünge 15, 15' des Bajonettverschlusses außer Eingriff sind und eine Abnahme des zweiten Gehäuseteils 2 ermöglichen.

Die in den Figuren dargestellte Vorrichtung weist eine elektronische Einrichtung 16 zur Erfassung der Abgaben des versprühbaren Stoffes auf. Diese umfasst dabei einen Schalter 17, der betätigt wird, wenn erster und zweiter Gehäuseteil zur Betätigung des Ventils 5 aufeinander zu bewegt werden.

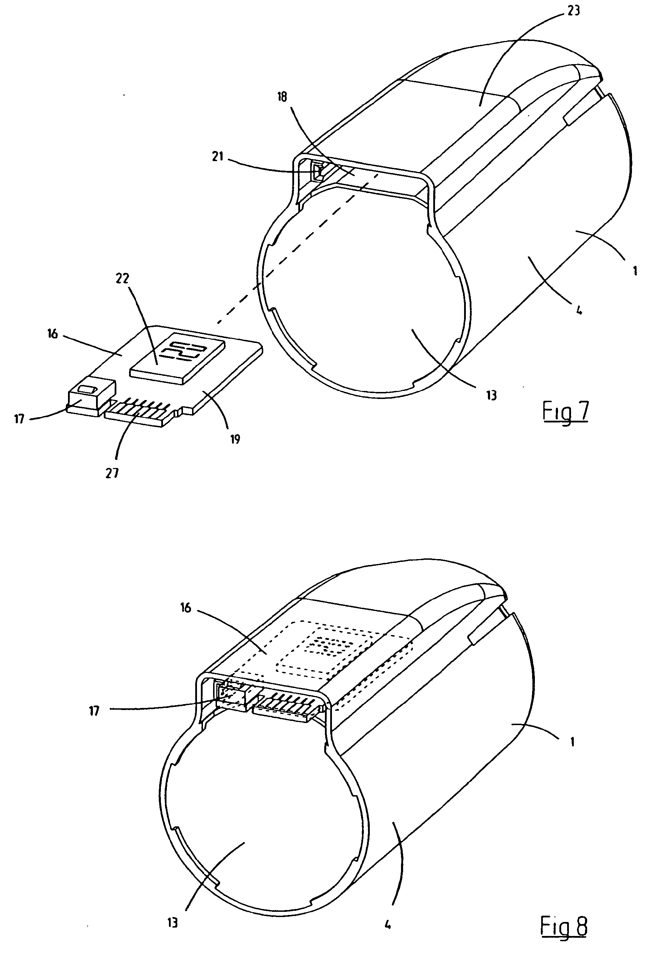

Wie z.B. in den Figuren 4 bis 9 dargestellt, wird die elektronische Einrichtung 16 in einer an dem ersten Gehäuseteil 1 ausgebildeten Halterung aufgenommen, welche bei der dargestellten Ausführungsform durch eine Kammer 18 mit im wesentlichen schlitzförmiger Gestalt gebildet wird. Die Kammer 18 schützt die elektronische Einrichtung 16 vor Beschädigungen und Manipulationen.

Die elektronische Einrichtung 16 weist dabei eine Platte 19 auf, welche als Leiterplatte bzw. Platine ausgebildet ist, um die erforderlichen Komponenten aufzunehmen und elektrisch leitend miteinander zu verbinden. Auch der Schalter 17 ist auf der Platte 19 angeordnet und weist einen elektrischen Kontakt auf, der beim Betätigen des Schalters geöffnet bzw. geschlossen wird. Die Platte 19 wird bei der dargestellten Ausführungsform durch Rastmittel 21 zuverlässig in der Kammer 18 gehalten (vgl. Fig. 9). Die Montage ist einfach, da die Platte 19 vorgefertigt werden kann und dann lediglich in die Kammer 18 eingesteckt werden muss. Alternativ kann die Platte 19 auch in die Kammer 18 eingegossen werden.

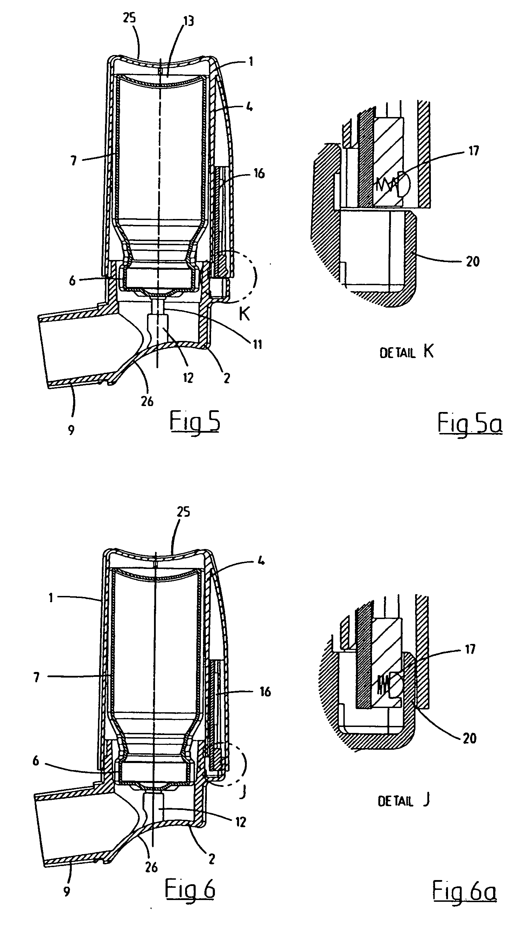

Der in den Figuren 5 bis 6a schematisch dargestellte Schalter 17 wird dabei durch ein Betätigungsabschnitt 20 betätigt, welcher an dem zweiten Gehäuseteil 2 angeordnet ist. Hier ist auch zu erkennen, dass der Schalter 17 an dem dem zweiten Gehäuseteil 2 zugewandten Endabschnitt des ersten Gehäuseteils 1 angeordnet ist. Der Schalter 17 liegt dabei nicht an dem Behälter 3 an. Vielmehr ist der Schalter 17 derart in der Kammer 18 des ersten Gehäuseteils angeordnet, dass der Schalter 17 geschützt liegt und nicht aus Versehen betätigt werden kann. Auch eine missbräuchliche Auslösung des Schalters 17 wird erschwert, da der Schalter 17 nicht mit einem Finger erreicht werden kann. Der vorstehende Betätigungsabschnitt 20 ist so an dem zweiten Gehäuseteil 2 angeordnet, dass er sich in die Kammer 18 hinein erstreckt, wenn erster und zweiter Gehäuseteil 1, 2 zur Betätigung des Ventils 5 aufeinander zu bewegt werden. Diese Stellung ist in den Figuren 6 und 6a dargestellt, während die Figuren 5, 5a die nicht betätigte Stellung zeigen.

Die elektronische Einrichtung 16 erfasst bei der dargestellten Ausführungsform die Anzahl der Abgaben bzw. der Betätigungen des Ventils 5. Hierzu weist die elektronische Einrichtung 16 auf der Platte 19 eine Recheneinheit, einen Datenspeicher und eine Energiequelle in Form einer Batterie auf (nicht im Einzelnen dargestellt), damit die durch den Schalter 17 erfassten Abgaben, bei denen jeweils ein elektrischer Kontakt geschlossen wird, gezählt werden können. Sofern in der elektronischen Einrichtung 16 vorab die maximale Anzahl der Betätigungen des Ventils 5 (entsprechend der jeweiligen Füllmenge des Behälters 3) hinterlegt wird, kann direkt die verbleibende Restmenge bzw. die verbleibende Anzahl an möglichen Abgaben des versprühbaren Stoffes ermittelt werden. Die jeweilige maximale Anzahl der Betätigungen kann durch Abspeicherung der entsprechenden Werte im Wege einer Programmierung oder aber mittels eines Kodierschalters oder dergleichen eingegeben werden.

Die ermittelte Information wird über eine kleine optische Anzeige 22, welche als LCD-Anzeige ausgebildet ist, angezeigt. Diese ist von außen sichtbar. Entsprechend ist die Abdeckung 23 der Kammer 18 aus einem durchsichtigen Material, insbesondere Kunststoff gefertigt. Alternativ oder zusätzlich zur optischen Anzeige kann ein nicht dargestellter akustischer Signalgeber vorgesehen werden.

Wenn die elektronische Einrichtung 16 über eine Uhr verfügt, sind weitergehende Funktionen möglich. In diesem Fall kann z.B. der Anwender optisch und/oder akustisch an die nächste Einnahme seines Medikaments erinnert werden. Weiterhin könnte dem Anwender der Ablauf des Haltbarkeitsdatums angezeigt oder ein entsprechender Warnhinweis gegeben werden. Praktisch ist auch ein Warnhinweis, sobald eine bestimmte Restmenge des versprühbaren Stoffes unterschritten wird. Zudem können die Einnahmezeitpunkte und die jeweiligen Dosierungen bzw. die Anzahl der abgegebenen Sprühstöße gespeichert werden.

Möglich ist es auch, die elektronische Einrichtung 16 so auszubilden, dass die gespeicherten Informationen über eine Schnittstelle ausgelesen werden können. In diesem Fall kann z.B. überprüft werden, wann und in welcher Dosierung das jeweilige Medikament eingenommen wurde. Hierzu sind an der Einrichtung 16 Kontakte 27 vorgesehen. Alternativ kann auch eine drahtlose Datenübertragung vorgesehen werden.

Wenn das Ventil 5 als Dosierventil ausgebildet ist, ist es nicht erforderlich, die Dauer der Betätigung des Ventils 5 zu erfassen, da mit jedem Sprühstoß jeweils eine gleichbleibende Menge des versprühbaren Stoffes abgegeben wird. Sofern jedoch ein einfaches Ventil verwendet wird, kann die Vorrichtung auch so ausgestaltet werden, dass sie die Dauer der Abgabe des versprühbaren Stoffes erfasst und damit auch in diesem Fall eine Ermittlung der abgegebenen Menge ermöglicht.

Erster und zweiter Gehäuseteil 1, 2 sind als Spritzgussteil aus Kunststoff gefertigt. Dabei ist der erste Gehäuseteil 1 aus durchsichtigem Kunststoff gefertigt, sodass nicht nur die optische Anzeige 22 sondern auch auf dem Behälter 3 angebrachte Informationen sichtbar sind.

Die dargestellte Vorrichtung weist eine nur geringe Anzahl an Bauteilen auf und ist kostengünstig herstellbar. Sie eignet sich daher auch für den Einmalgebrauch. Einmalgebrauch in diesem Sinne liegt dann vor, wenn die Vorrichtung gleichzeitig mit dem jeweiligen Behälter erneuert wird. Selbstverständlich wäre auch eine mehrmalige Verwendung möglich. In diesem Fall ist, z.B. über einen Reset-Schalter, dafür Sorge zu tragen, dass mit Erneuerung des Behälters die elektronische Einrichtung 16 zurück auf den Ursprungszustand gesetzt wird.

### Bezugszeichenliste

- 1: erster Gehäuseteil
- 2: zweiter Gehäuseteil
- 3: Behälter
- 4: Schaft
- 5: Ventil
- 6: Behälterkopf
- 7: Behälterkörper
- 8: erster Abschnitt
- 9: zweiter Abschnitt
- 10: Kappe
- 11: Ventilstift
- 12: Betätigungsabschnitt
- 13: Aufnahme
- 14, 14': Anschläge
- 15, 15': Vorsprünge
- 16: Einrichtung zur Erfassung der Abgaben
- 17: Schalter
- 18: Kammer
- 19: Platte
- 20: Betätigungselement
- 21: Rastmittel
- 22: optische Anzeige
- 23: Abdeckung
- 24: Bodenabschnitt
- 25: erster Griffabschnitt
- 26: zweiter Griffabschnitt
- 27: Kontakte
- 28: Bohrung

## Patentansprüche

1. Vorrichtung zur dosierten Abgabe von versprühbaren Stoffen, insbesondere von Aerosolen, mit einer elektronischen Einrichtung (16) zur Erfassung der Abgaben, und mit einem ersten Gehäuseteil (1), der eine Aufnahme (13) für einen den versprühbaren Stoff enthaltenden mit einem Ventil (5) versehenen Behälter (3) bildet, **gekennzeichnet durch** einen einen Betätigungsabschnitt (29) zur Einwirkung auf das Ventil (5) aufweisenden zweiten Gehäuseteil (2), der relativ zu dem ersten Gehäuseteil (1) bewegbar ist, um das Ventil (5) zur Abgabe des versprühbaren Stoffes zu betätigen, wobei die elektronische Einrichtung (16) zur Erfassung der Abgaben des versprühbaren Stoffes einen Schalter (17) aufweist, der **durch** die Relativbewegung des ersten und des den Betätigungsabschnitt zur Einwirkung auf das Ventil (5) aufweisenden zweiten Gehäuseteils (1, 2) betätigt wird, wobei der erste und der zweite Gehäuseteil (1, 2) derart verbunden sind, dass erster und zweiter Gehäuseteil (1, 2) zueinander eine Arbeitsposition, in der der erste und zweite Gehäuseteil (1, 2) relativ zueinander in axialer Richtung beweglich sind, um das Ventil zur Abgabe des versprühbaren Stoffes zu betätigen, und eine Freigabeposition einnehmen können, wobei in der Freigabeposition, in der der zweite Gehäuseteil von dem ersten Gehäuseteil abnehmbar ist, erster und zweiter Gehäuseteil (1, 2) nicht derart axial zueinander beweglich sind, dass das Ventil (5) **durch** den zweiten Gehäuseteil (2) betätigt werden kann.

2. Vorrichtung nach Anspruch 1, **dadurch gekennzeichnet, dass** der erste und der zweite Gehäuseteil (1, 2) über einen Bajonettverschluss verbunden sind.

3. Vorrichtung nach Anspruch 1 oder 2, **dadurch gekennzeichnet, dass** an erstem und zweiten Gehäuseteil (1,2) Anschläge (14, 14') vorgesehen sind, welche die axiale Bewegung des ersten und zweiten Gehäuseteils (1,2) aufeinander zu nur in der Arbeitsposition gestatten.

4. Vorrichtung nach einem der Ansprüche 1 bis 3, **dadurch gekennzeichnet, dass** der Schalter (17) ein mechanischer Schalter, ein Näherungsschalter, ein Magnetschalter, und/oder ein optischer Schalter ist.

5. Vorrichtung nach einem der Ansprüche 1 bis 4, **dadurch gekennzeichnet, dass** der Schalter (17) nicht zur Anlage an dem Behälter (3) ausgebildet ist.

6. Vorrichtung nach einem der Ansprüche 1 bis 5, **dadurch gekennzeichnet, dass** in der Aufnahme (13) der Behälter (3), welcher einen Behälterkörper (7) und einen Behälterkopf (6) mit dem Ventil (5) zur dosierten Abgabe des versprühbaren Stoffes aufweist, aufnehmbar ist.

7. Vorrichtung nach Anspruch 6, **dadurch gekennzeichnet, dass** der erste Gehäuseteil (1) einen hohlen Schaft (4) aufweist, in dem der Behälterkörper (7) ganz oder teilweise aufnehmbar ist und/oder dass der zweite Gehäuseteil (2) einen ersten Abschnitt (8) zur Aufnahme des Behälterkopfes (6) und einen insbesondere winklig zu dem ersten Abschnitt (8) verlaufenden hohlen zweiten Abschnitt (9) aufweist.

8. Vorrichtung nach einem der Ansprüche 1 bis 7, **dadurch gekennzeichnet, dass** der zweite Gehäuseteil (2) abnehmbar ist.

9. Vorrichtung nach einem der Ansprüche 1 bis 8, **dadurch gekennzeichnet, dass** durch die elektronische Einrichtung (16) zur Erfassung der Abgaben die Anzahl und/oder die Dauer der Abgaben erfasst wird.

10. Vorrichtung nach einem der Ansprüche 1 bis 9, **dadurch gekennzeichnet, dass** die elektronische Einrichtung (16) zur Erfassung der Abgaben eine elektronische Recheneinheit und einen elektronischen Datenspeicher aufweist, in denen die durch den Schalter (17) erfassten Abgaben gezählt werden können.

11. Vorrichtung nach einem der Ansprüche 1 bis 10, **dadurch gekennzeichnet, dass** die elektronische Einrichtung (16) zur Erfassung der Abgaben mit dem Schalter (17) an oder in dem ersten oder zweiten Gehäuseteil (1, 2) angeordnet ist, und dass der Schalter (17) durch ein an dem jeweils anderen Gehäuseteil (2, 1) vorgesehenes Betätigungselement (20) bei einer Relativbewegung von erstem und zweitem Gehäuseteil (1, 2) betätigt wird.

12. Vorrichtung nach einem der Ansprüche 1 bis 11, **dadurch gekennzeichnet, dass** die Vorrichtung als Inhalationsvorrichtung insbesondere für ein Medikament ausgebildet ist.

13. Vorrichtung nach einem der Ansprüche 1 bis 12, **dadurch gekennzeichnet, dass** in der Aufnahme (13) ein mit dem versprühbaren Stoff wenigstens teilweise befüllter Behälter (3) aufgenommen ist.

14. Vorrichtung nach einem der Ansprüche 1 bis 13, **dadurch gekennzeichnet, dass** der versprühbare Stoff ein Medikament ist.

15. Vorrichtung nach einem der Ansprüche 1 bis 14, **dadurch gekennzeichnet, dass** das Ventil (5) des Behälters (3) als ein Dosierventil ausgebildet ist, welches bei jeder Betätigung eine gleichbleibende Menge des versprühbaren Stoffes insbesondere stoßartig abgibt.

## Claims

1. Apparatus for dispensing sprayable substances, in particular aerosols, in a metered manner, having an electronic device (16) for detecting the dispensing operations, and having a first housing part (1) which forms a receptacle (13) for a container (3) which contains the sprayable substance and is provided with a valve (5), **characterized by** a second housing part (2) which has an operating section (29) for acting on the valve (5) and which can be moved relative to the first housing part (1) in order to operate the valve (5) for dispensing the sprayable substance, with the electronic device (16) for detecting the dispensing operations of the sprayable substance having a switch (17) which is operated by the relative movement of the first and of the second housing part (1, 2) which has the operating section for acting on the valve (5), with the first and the second housing part (1, 2) being connected in such a way that the first and the second housing part (1, 2) can assume, relative to one another, a working position, in which the first and second housing parts (1, 2) can be moved relative to one another in the axial direction in order to operate the valve for the purpose of dispensing the sprayable substance, and a release position, it not being possible to move the first and the second housing part (1, 2) relative to one another in an axial manner in such a way that the valve (5) can be operated by the second housing part (2) in the release position in which the second housing part can be removed from the first housing part.

2. Apparatus according to Claim 1, **characterized in that** the first and the second housing part (1, 2) are connected by means of a bayonet fitting.

3. Apparatus according to Claim 1 or 2, **characterized in that** stops (14, 14') are provided on the first and second housing parts (1, 2), the said stops allowing the axial movement of the first and second housing parts (1, 2) towards one another only in the working position.

4. Apparatus according to one of Claims 1 to 3, **characterized in that** the switch (17) is a mechanical switch, a proximity switch, a magnetic switch and/or an optical switch.

5. Apparatus according to one of Claims 1 to 4, **characterized in that** the switch (17) is not designed to bear against the container (3).

6. Apparatus according to one of Claims 1 to 5, **characterized in that** the container (3), which has a container body (7) and a container head (6) having the valve (5) for dispensing the sprayable substance in a metered manner, can be accommodated in the receptacle (13).

7. Apparatus according to Claim 6, **characterized in that** the first housing part (1) has a hollow shaft (4) in which the container body (7) can be fully or partially accommodated and/or **in that** the second housing part (2) has a first section (8) for accommodating the container head (6) and a hollow second section (9) which runs, in particular, at an angle to the first section (8).

8. Apparatus according to one of Claims 1 to 7, **characterized in that** the second housing part (2) can be removed.

9. Apparatus according to one of Claims 1 to 8, **characterized in that** the number and/or the duration of dispensing operations is detected by the electronic device (16) for detecting the dispensing operations.

10. Apparatus according to one of Claims 1 to 9, **characterized in that** the electronic device (16) for detecting the dispensing operations has an electronic computer unit and an electronic data storage unit, in which units the dispensing operations which are detected by the switch (17) can be counted.

11. Apparatus according to one of Claims 1 to 10, **characterized in that** the electronic device (16) for detecting the dispensing operations with the switch (17) is arranged on or in the first or second housing part (1, 2), and **in that** the switch (17) is operated by an operating element (20), which is provided on the respectively other housing part (2, 1), in the event of a relative movement of the first and second housing parts (1, 2).

12. Apparatus according to one of Claims 1 to 11, **characterized in that** the apparatus is in the form of an inhalation apparatus, in particular for a medicament.

13. Apparatus according to one of Claims 1 to 12, **characterized in that** a container (3) which is at least partially filled with the sprayable substance is accommodated in the receptacle (13).

14. Apparatus according to one of Claims 1 to 13, **characterized in that** the sprayable substance is a medicament.

15. Apparatus according to one of Claims 1 to 14, **characterized in that** the valve (5) of the container (3) is in the form of a metering valve which dispenses a constant quantity of the sprayable substance, in particular in a burst, each time it is operated.

## Revendications

1. Ensemble pour délivrer de manière dosée des substances pulvérisables, en particulier des aérosols, l'ensemble présentant un dispositif électronique (16) qui saisit les quantités émises et une première partie (1) de boîtier qui forme un logement (13) pour un récipient (3) doté d'une soupape (5) et contenant la substance pulvérisable,
**caractérisé par**
une deuxième partie (2) de boîtier qui présente une partie d'actionnement (29) qui agit sur la soupape (5) et qui peut être déplacée par rapport à la première partie (1) de boîtier pour actionner la soupape (5) en vue de délivrer la substance pulvérisable,
le dispositif électronique (16) présentant pour saisir les quantités émises de substance pulvérisable un commutateur (17) qui est actionné par le déplacement relatif entre la première partie (1) de boîtier et la deuxième partie (2) de boîtier qui présente la partie d'actionnement qui agit sur la soupape (5),
la première et la deuxième partie (1, 2) de boîtier étant reliées l'une à l'autre de telle sorte que la première et la deuxième partie (1, 2) de boîtier puissent prendre l'une par rapport à l'autre une position de travail dans laquelle la première et la deuxième partie (1, 2) de boîtier peuvent être déplacées l'une par rapport à l'autre dans la direction axiale pour actionner la soupape pour qu'elle délivre la substance pulvérisable et une position de libération,
la première et la deuxième partie (1, 2) de boîtier ne pouvant pas être déplacées axialement l'une par rapport à l'autre pour que la soupape (5) puisse être actionnée par la deuxième partie de boîtier (2) dans la position de libération dans laquelle la deuxième partie de boîtier peut être retirée de la première partie de boîtier.

2. Ensemble selon la revendication 1, **caractérisé en ce que** la première et la deuxième partie (1, 2) de boîtier sont reliées par une fermeture à baïonnette.

3. Ensemble selon les revendications 1 ou 2, **caractérisé en ce que** des butées (14, 14') qui ne permettent le déplacement axial de la première et la deuxième partie de boîtier (1, 2) que dans la position de travail sont prévues sur la première et la deuxième partie (1, 2) de boîtier.

4. Ensemble selon l'une des revendications 1 à 3, **caractérisé en ce que** le commutateur (17) est un commutateur mécanique, un commutateur de proximité, un commutateur magnétique et/ou un commutateur optique.

5. Ensemble selon l'une des revendications 1 à 4, **caractérisé en ce que** le commutateur (17) n'est pas configuré pour venir se placer sur le récipient (3).

6. Ensemble selon l'une des revendications 1 à 5, **caractérisé en ce que** le récipient (3) qui présente un corps de récipient (7) et une tête de récipient (6) qui présente la vanne (5) qui libère de manière dosée la substance pulvérisable peut être logé dans le logement (13).

7. Ensemble selon la revendication 6, **caractérisé en ce que** la première partie (1) de boîtier présente une tige creuse (4) dans laquelle le corps (7) du récipient peut être logé en tout ou en partie et/ou **en ce que** la deuxième partie (2) de boîtier présente une première partie (8) qui loge la tête (6) du récipient et une deuxième partie (9) creuse qui s'étend en particulier obliquement par rapport à la première partie (8).

8. Ensemble selon l'une des revendications 1 à 7, **caractérisé en ce que** la deuxième partie (2) du boîtier est amovible.

9. Ensemble selon l'une des revendications 1 à 8, **caractérisé en ce que** le nombre et/ou la durée des émissions sont détectés par le dispositif électronique (16) pour saisir les quantités émises.

10. Ensemble selon l'une des revendications 1 à 9, **caractérisé en ce que** pour saisir les quantités émises, le dispositif électronique (16) présente une unité électronique de calcul et une mémoire électronique de données dans lesquelles les émissions saisies par le commutateur (17) peuvent être comptées.

11. Ensemble selon l'une des revendications 1 à 10, **caractérisé en ce que** pour saisir les émissions, le dispositif électronique (16) est disposé avec le commutateur (17) sur ou dans la première ou la deuxième partie (1, 2) de boîtier et **en ce que** le commutateur (17) est actionné par un élément d'actionnement (20) prévu sur l'autre partie (2, 1) de boîtier en cas de déplacement relatif entre la première et la deuxième partie (1, 2) de boîtier.

12. Ensemble selon l'une des revendications 1 à 11, **caractérisé en ce que** l'ensemble est configuré comme ensemble d'inhalation, en particulier d'un médicament.

13. Ensemble selon l'une des revendications 1 à 12, **caractérisé en ce qu'**un récipient (3) rempli au moins en partie de la substance pulvérisable est logé dans le logement (13).

14. Ensemble selon l'une des revendications 1 à 13, **caractérisé en ce que** la substance pulvérisable est un médicament.

15. Ensemble selon l'une des revendications 1 à 14, **caractérisé en ce que** la soupape (5) du récipient (3) est configurée comme soupape de dosage qui, lors de chaque actionnement, délivre en particulier brusquement une quantité constante de substance pulvérisable.
